# EUROPEAN PATENT SPECIFICATION

(11) **EP 2 499 980 B1**
(45) Date of publication and mention of the grant of the patent: **30.10.2013**
(21) Application number: 12158717.4
(22) Date of filing: 09.03.2012
(51) Int. Cl.: A61B 17/54

(54) **Pedicure device with interchangeable cutting blade**
Fußpflege-Einrichtung mit auswechselbarer Schneidklinge
Dispositif de pédicure avec lame de coupage interchangeable

(30) Priority: 16.03.2011 IT VI20110021 U
(43) Date of publication of application: 19.09.2012
(73) Proprietor: Palmieri, Giuseppe, 36040 Torri di Quartesolo (VI) (IT); Valerio, Andrea, 36010 Monticello Conte Otto (VI) (IT); Valerio, Fabio, 36010 Monticello Conte Otto (IT)
(72) Inventor: Palmieri, Giuseppe, 36040 Torri di Quartesolo (VI) (IT); Valerio, Andrea, 36010 Monticello Conte Otto (VI) (IT); Valerio, Fabio, 36010 Monticello Conte Otto (IT)
(74) Representative: Bettello, Pietro

(56) References cited:
- WO-A2-2006/006036
- DE-A1- 3 724 912
- US-A- 2 425 665
- US-A- 2 431 118
- US-A- 2 619 724
- US-A- 3 187 431
- US-A- 3 367 727
- US-A- 5 924 206

## Description

The present invention relates to a pedicure device with an interchangeable cutting blade.

It is known that devices provided with a handle which, at one of the ends thereof, have an interchangeable cutting blade through which the operator carries out pedicure operations are available in the market.

In the state of the art, the utility model n° 64279/B/81 is present, which describes a cylindrical handle, provided - at an end thereof - with a conical clamp, comprising a fixed jaw and a mobile jaw. Between the aforementioned a cutting blade is arranged; the closure of the clamp occurs by screwing a threaded pin, integral with the mobile jaw, arranged within a threaded blind hole provided for on a rod which actually constitutes the handle of the device.

At the time, such device had the advantage, with respect to the similar devices of the known type, of allowing a simpler replacement of the cutting blade. Actually, it should be borne in mind that, due to evident hygiene reasons, the operator is required to replace the cutting blade, even though it is still capable of serving its purpose efficiently, upon changing the client on which it operates. Furthermore, the operator - at times even when operating on the same client - is required to change the blade all the same in case of operation in body areas or on skin thicknesses of different types, which require the use of blades of different sizes.

Actually, the operation of replacing the blade of such device, though it can be performed in a much easier and quicker manner with respect to similar devices of the known type, is however not extremely quick. Actually, it should be considered that - very often - such manoeuvre is carried out by the operator wearing protection gloves, for obvious hygiene reasons; in particular, the operator would desire to perform the blade replacement operation using only one hand, while the other is occupied supporting the sealed casing from which a new sterilised blade to be used is removed. Other important documents of the state of the art are: WO 2006/006036, US 2619724, US 2431118, US 5924206, US 2425665 and US 3187431. Document WO 2006/006036 discloses a pedicure device with interchangeable cutting blades actuated by pushing a button at a proximal end of the device.

An object of the present model is that of providing a device of the type described above, which allows replacing the blade and disassamble the device for cleaning and sterilisation purposes in a much simpler and quicker manner with respect to the manner that can be carried out in the device indicated above, as well as in all other similar devices of the known type.

This is obtained by the pedicure device defined in appended claim 1, with preferred embodiments as defined in the appended dependent claims 2 to 6.

The model will be now described in detail with reference to some particular embodiments thereof, provided by way of non-limiting example, with reference to the attached drawings, wherein;
- figs 1 and 2 illustrate, respectively, a perspective view of the device according to the invention in conditions of actual operation and an exploded perspective view of the aforementioned;
- fig 3 shows a longitudinal sectional view of the device according to the model;

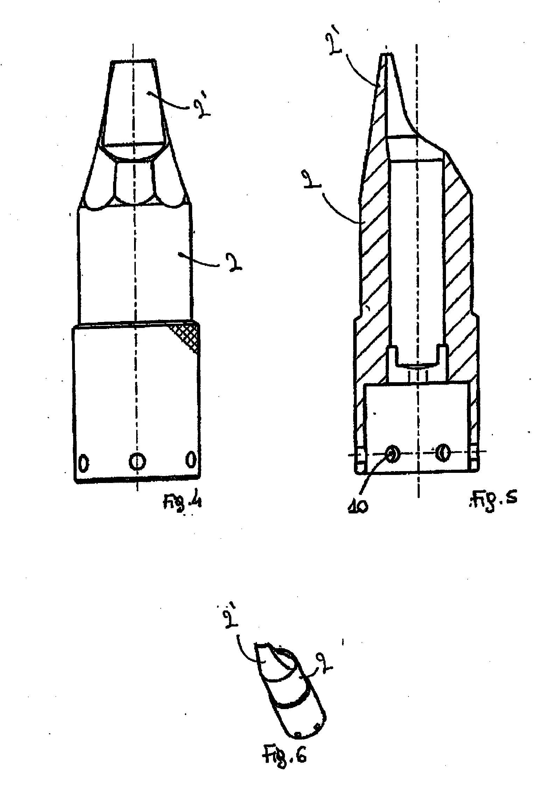

- figs 4, 5 and 6 illustrate, respectively, a lateral view, a longitudinal sectional view and a perspective view of a first embodiment of the clamp which holds the cutting blade;

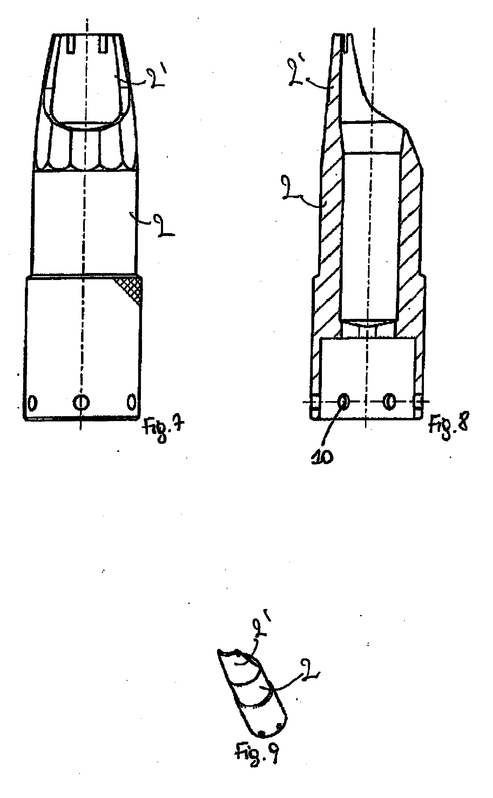

- figs 7, 8 and 9 illustrate, respectively, a lateral view, a longitudinal section view and a perspective view of a second embodiment of the fixed jaw which holds the cutting blade;

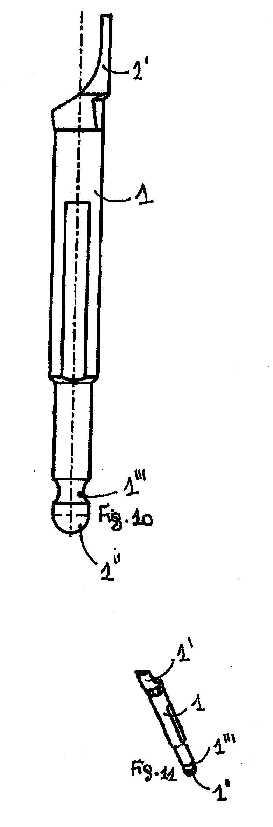

- figs 10 and 11 illustrate, respectively, a lateral view and perspective view of a first embodiment of the mobile jaw which holds the cutting blade, in collaboration with the device illustrated in figures 4 to 6;

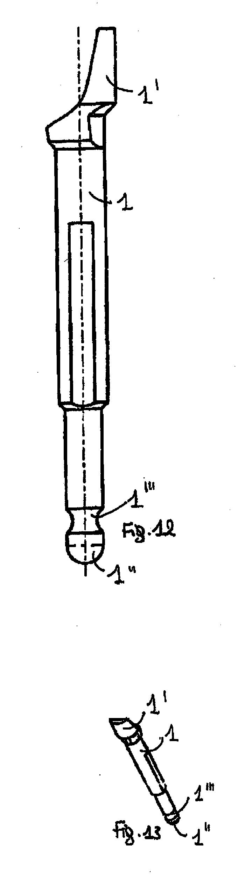

- figs 12 and 13 illustrate, respectively, a lateral view and a perspective view of a further embodiment of the mobile jaw, adapted to collaborate with the fixed jaw illustrated in figures 7 to 9;

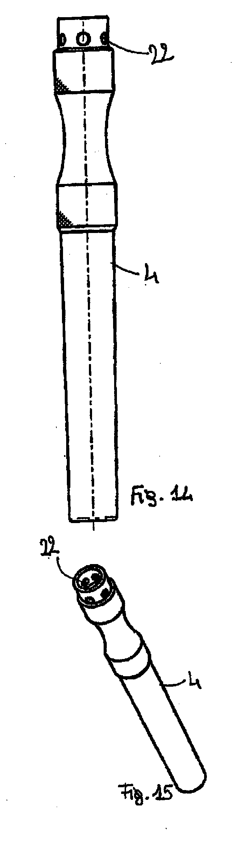

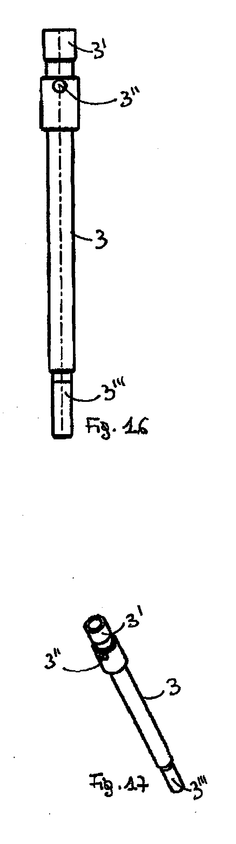

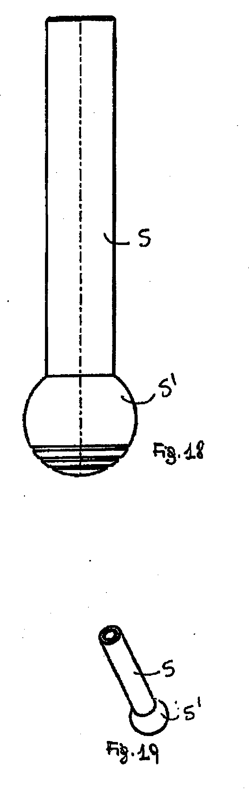

- figures 14 and 15, 16 and 17 as well as 18 and 19 respectively illustrate a lateral view and a perspective view of three of the elements present in the device subject of the invention.

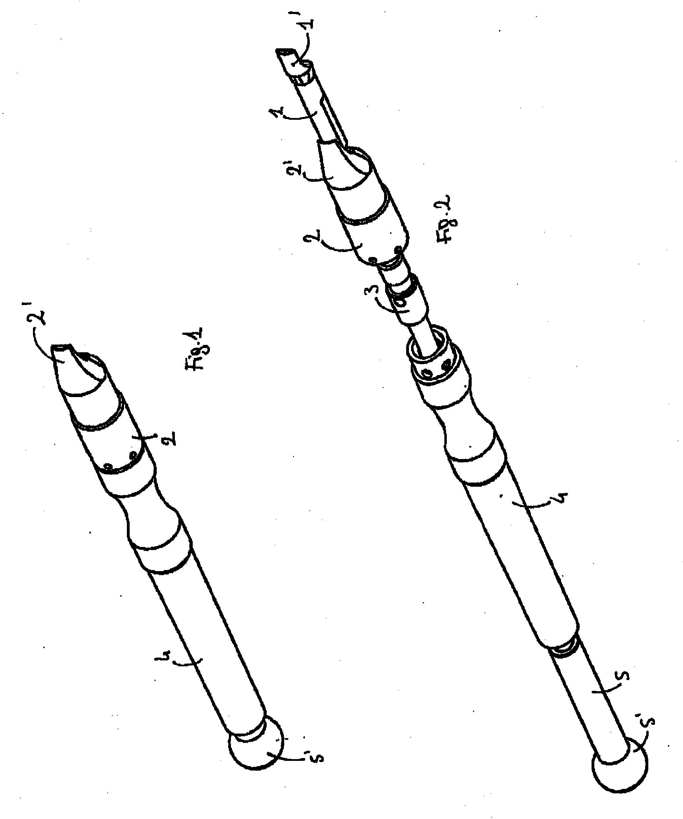

As observable in figures 1 and 2, the pedicure device according to the invention is of the type showing a handle, which - at one of the ends thereof - has a clamp-like structure between whose jaws the cutting blade is constrained. The device according to the invention is further characterised by the configuration of the component parts thereof, which allow, through a simple manoeuvre, to free the cutting blade from the constraint between the two jaws, due to the reasons exhaustively explained above.

In particular, figs 1-3 show that the clamp present in the device shows a first jaw, consisting of a projecting and tapered end 2' of a spout 2, within which a pin 1 is arranged, whose end 1' has a shape conjugated with that of the end 2' of the spout, which serves as a second jaw of the clamp.

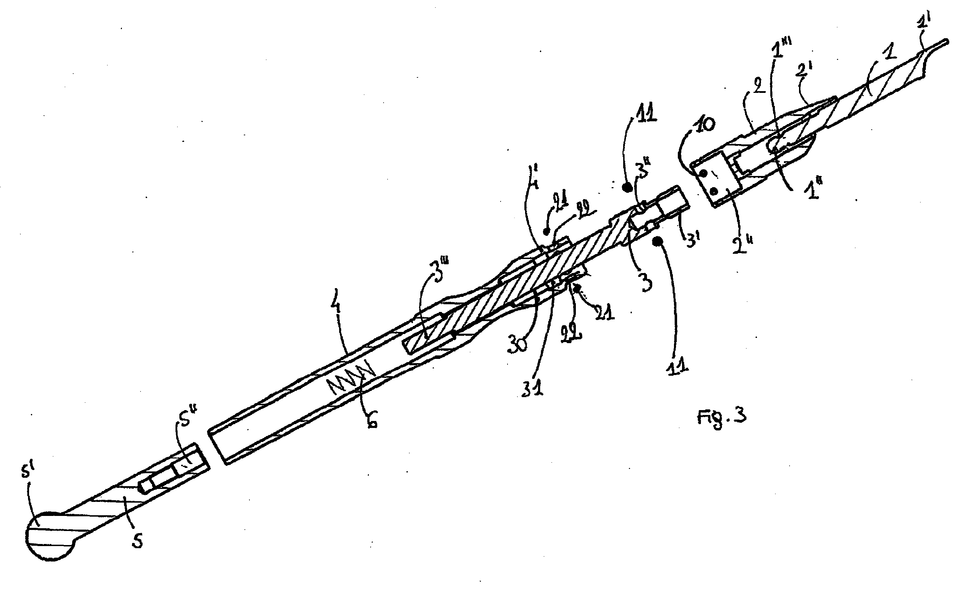

As observable in particular in the sectional view of figure 3, the end 1" of the pin 1, opposite to the tip-shaped end 1', is configured sphere-like and it is characterised by a collar-shaped area 1"'; such end 1'" is adapted to be inserted into a seat 3' obtained on the end of a shaft 3, which (in turn) can be inserted into the central cavity of a substantially cylindrical-shaped handle 4. At the side wall of the seat 3' two holes 3" are present, into each one of which a sphere 11 is inserted, which has a given freedom of motion within said hole. Should the end 1" be inserted into the seat 3', up to ensuring that the sphere 11 ends up at the annular hole 1'" of the pin 1, the fixing of the latter with the shaft occurs, under the conditions to be explained further hereinafter.

Also the spout 2 can be fixed to the handle 4 and in order to allow this fixing three further spheres 21 are present, inserted into special holes 22 present at the end 4' of the handle 4, facing towards the area of the tool in which the blade is present.

When the spout 2, is brought to contact with the handle 4, the insertion of the outer surface end of the spheres 21 into the holes 10 present at the end of the spout occurs, determining (also in this case) the fixing between these two elements.

The end 3'" of the shaft arranged at the rear part is threaded and around the aforementioned a coil spring 6. is arranged Such threaded end 3'" is adapted to be engaged by screwing into a cavity 5", also threaded, present in the button 5, which, advantageously, has an end 5' having a widened shape, so as to facilitate the user when pressing the button.

When the device is in the actual utilisation mounted state (fig 1) the various spheres, alongside the other solutions described previously, determine the stable mutual positioning in fixed position of the various elements constituting the device and, in particular, the stable positioning of the cutting blade between the jaws 1' and 2'.

Vice versa, should the user desire to eliminate the cutting blade (possibly for replacing it with another) one may slightly press the button 5, through the widened end 5'. Such manoeuvre determines a compression of the spring 6 and thus the displacement towards the front part of the shaft 3, ensuing forward movement of the pin 1 with respect to the spout 2. In this manner, the blade is freed from the constraint between the two jaws of the clamp and it can thus be removed from the device.

Should the operator desire to insert another blade into the device, the operator shall be simply required to partly press the button and thus arrange the blade in its seat. Vice versa, should the operator no longer intend to use the device and intend to sterilise it, the operator shall be required to fully press the button 5.

Under normal operating conditions of the device, the spheres 11 compress the collar 1 "' of the pin 1, due to the contact thereof with the narrower surface 30 of the seat within the handle 4. Vice versa upon fully pressing the button 5, the partial exit of the shaft 3 from the handle 4 occurs, so that the spheres 21 end up arranged in an area in which they end up at contact with a wider surface of such seat.

This determines the release of the pin 1 from the shaft 3, due to the fact that the spheres 11 project out of the seat of the collar 1"'; at the same time the release of the spout 2 from the handle 4 also occurs, due to the disengagement of the spheres 21 from the holes 10.

This determines the partial exit of the shaft 3 from the handle 4, from a position in which the spheres 11 compress the collar 1"' of the pin 1, due to the contact thereof with the surface 30 having a narrower diameter than the inner diameter of the handle 4. Due to such exit, the spheres 11 end up in an area in which they end up in contact with a surface 31 having a diameter larger than such seat.

According to the information above, it can be observed that the clamping modes (by the spheres 11 and 21) are opposite. Actually, the spheres 11 fasten the clamp 1 through a compression action on the collar 1"', while the spheres 21 block the spout 2 operating in an external thrust being inserted into the holes 10 present in the spout 2.

Those described above are the manoeuvres that can be performed by the operator, during use, and to allow the sterilisation of the device.

The ordinary maintenance of the device, which implies the total demounting of the aforementioned, is (vice versa) intended to be performed by assistance centres, which will provide for also mutually separating the various elements of the rear part of the device (in practice, the button 5, the handle 4 and the shaft 3), which generally remain always fixed mutually, though having - in any case - a given mutual sliding degree, due to the presence of the coil spring 6.

From what has been described above, it can thus be observed that with the device according to the invention both the operation of replacing a cutting blade and that of ordinary maintenance can be performed in an extremely simple manner that can be carried out by any operator. All these operations can be carried out in an extremely simple manner even by a person not particularly skilled in the mechanism, substantially using only one hand, which can be covered with a protection glove, the entire operation being performed without any difficulty.

## Claims

1. Pedicure device with interchangeable cutting blades comprising:
a substantially cylindrical-shaped handle (4);
a clamp structure detachably coupled at one end of the handle (4), the clamp structure comprising a first jaw (2) and a second jaw (1) for gripping a cutting blade;
a shaft (3) adapted to be inserted into a central cavity of the handle (4), the shaft (3) comprising a seat (3') at one of its ends; and
an actuator button (5) located at the opposite end of the handle (4) where the clamp structure is located, the actuator button (5) being adapted to be coupled to shaft (3),
wherein the first jaw (2) comprises a projecting and tapered end (2') and a spout (2),
wherein the second jaw (1) has a pin-like structure comprising at one end thereof a conjugate shape (1') to that of the projecting and tapered end (2') of the first jaw (2) and at the other end a sphere-like shape end (1 ") and a collar-shaped area (1'"),
wherein the end of the second jaw (1) that comprises the sphere-like end (1") and the collar-shaped area (1"') is adapted to be inserted into the seat (3') of the shaft (3);
wherein a slight pressing action on the button (5) results in the opening of the clamp structure for permitting a cutting blade change; and
wherein an increased pressing action on the button (5) results in the release of the first jaw (2) and the second jaw (1) from the handle (4) for permitting their cleaning and sterilisation.

2. DEVICE, according to claim 1, **characterised in that** at the side wall of the seat (3') two holes are present (3"'), into each one of which there is inserted a sphere (11) which has a given freedom of movement within the hole into which it is inserted, it being provided for that, when the end (1") of the pin (1) is inserted into the seat (3) it occurs that the spheres (21) end up at the annular groove (1"') of the pin 1, determining the fastening of the latter with the shaft (3).

3. DEVICE, according to claim 2, **characterised in that** the spout (2) can be fastened to the handle (4) and, in order to allow this fastening, three further spheres (21) are present, inserted into holes (22) present at the end (4') of the handle (4) facing towards the area of the tool in which a blade is present, it being provided for that when the spout (2) is brought to contact with the handle (4) the insertion of the outer surface end of the spheres (11) into the holes (10) present at the end of the spout occurs, thus determining the fastening between these two elements.

4. DEVICE, according to claim 4, **characterised in that** the end (3"') of the shaft arranged at the rear part is threaded and around the aforementioned a coil spring is arranged (6), said threaded end (3"') being adapted to be engaged by screwing into a cavity (5"), also threaded, present in the button (5), which has a widened-shaped outer end (5').

5. DEVICE, according to claim 4, **characterised in that** upon slightly pressing the button (5) the compression of the spring (6) and thus the displacement towards the front part of the shaft (3) occurs, ensuing forward movement of the pin (1) with respect to the spout (2), thus allowing the blade to be released from the constraint between the two jaws of the clamp.

6. DEVICE, according to claim 5, **characterised in that** in use the spheres (11) compress the collar (1"') of the pin (1), due to the contact thereof with a recessed surface (30) on the cavity of the handle (4) which has a narrower diameter than the inner seat of the handle (4), wherein upon increased pressing action on the button (5) the partial exit of the shaft (3) from the handle (4) occurs, so that the spheres (11) end up in an area wherein they get in contact with a surface (31) having a diameter wider than such seat, determining the release of the pin (1) from the shaft (3), due to the fact that the spheres (11) exit from the seat of the collar (1"') and simultaneously determining also the release of the spout (2) from the handle (4), due to the disengagement of the spheres (21) from the holes (10).

## Patentansprüche

1. FUßPFLEGE-EINRICHTUNG MIT AUSWECHSELBAREREN SCHNEIDKLINGEN, umfassend: einen im Wesentlichen zylinderförmigen Griff (4); eine lösbar mit einem Ende des Griffs (4) verbundene Einspannanordnung, wobei die Einspannanordnung eine erste Backe (2) und eine zweite Backe (1) zum Einspannen einer Schneidklinge umfasst; einen Schaft (3), der geeignet ist, in einen zentralen Hohlraum des Griffs (4) eingefügt zu werden, wobei der Schaft (3) eine Aufnahme (3') an einem seiner Enden umfasst; und einen Betätigungsknopf (5), der sich an dem Ende des Griffs (4), befindet, das dem entgegengesetzt ist, wo sich die Einspannanordnung befindet, wobei der Betätigungsknopf (5) geeignet ist, mit dem Schaft (3) verbunden zu werden, wobei die erste Backe (2) ein vorstehendes und sich verjüngendes Ende (2') und eine Tülle (2) umfasst, wobei die zweite Backe (1) einen stiftartigen Aufbau aufweist, der an einem Ende desselben eine der Form des vorstehenden und sich verjüngenden Endes (2') der ersten Backe (2) angepasste Form (1') und an dem anderen Ende ein kugelförmiges Endstück (1") und einen bundförmigen Bereich (1'") umfasst, wobei das Ende der zweiten Backe (1), das das kugelförmige Endstück (1") und den bundförmigen Bereich (1"') umfasst, geeignet ist, in die Aufnahme (3') des Schafts (3) eingefügt zu werden; wobei eine geringfügige Druckwirkung auf den Knopf (5) zur Folge hat, dass sich die Einspannanordnung öffnet, um den Schneidklingenwechsel zu ermöglichen; und wobei eine stärkere Druckwirkung auf den Knopf (5) zur Folge hat, dass sich die erste Backe (2) und die zweite Backe (1) vom Griff (4) lösen, um ihre Reinigung und Sterilisation zu ermöglichen.

2. EINRICHTUNG nach Anspruch 1, **dadurch gekennzeichnet, dass** sich in der Seitenwand der Aufnahme (3') zwei Löcher befinden (3"'), in jedes von denen eine Kugel (11) eingesetzt ist, die eine gegebene Bewegungsfreiheit innerhalb des Lochs, in das sie eingesetzt ist, hat, wobei vorgesehen ist, dass, wenn das Endstück (1") des Stifts (1) in die Aufnahme (3) eingefügt ist, die Kugeln (21) in die ringförmige Nut (1"') des Stifts 1 gelangen und die Befestigung dieses Letzteren an dem Schaft (3) bedingen.

3. EINRICHTUNG nach Anspruch 2, **dadurch gekennzeichnet, dass** die Tülle (2) an dem Griff (4) befestigt werden kann, und dass, um dieses Befestigen zu ermöglichen, drei weitere Kugeln (21) vorhanden sind, die in Löcher (22) eingesetzt sind, die sich an dem Ende (4') des Griffs (4) befinden, das der Fläche des Werkzeugs zugewandt ist, in dem sich eine Klinge befindet, wobei vorgesehen ist, dass sich, wenn die Tülle (2) in Kontakt mit dem (4) gebracht wird, das Außenflächenende der Kugeln (11) in die an dem Ende der Tülle befindlichen Löcher (10) einfügt und so die Befestigung zwischen diesen zwei Elementen bedingt.

4. EINRICHTUNG nach Anspruch 4, **dadurch gekennzeichnet, dass** das am hinteren Teil angeordnete Ende (3'") des Schafts mit einem Gewinde versehen ist, und dass um das Vorgenannte eine Spiralfeder (6) angeordnet ist, wobei das mit dem Gewinde versehene Ende (3"') geeignet ist, durch Schrauben in den ebenfalls mit einem Gewinde versehenen Hohlraum (5") in Eingriff gebracht zu werden, der sich in dem Knopf (5) befindet, der ein äußeres Ende (5') mit einer aufgeweiteten Form hat.

5. EINRICHTUNG nach Anspruch 4, **dadurch gekennzeichnet, dass** bei leichtem Drücken des Knopfes (5) die Kompression der Feder (6) und folglich die Verschiebung zum vorderen Teil des Schafts (3) hin eintreten, woraus die Vorwärtsbewegung des Stifts (1) gegenüber der Tülle (2) folgt, wodurch es möglich wird, dass die Klinge aus der Zurückhaltung zwischen den zwei Backen der Einspannung gelöst wird.

6. GERÄT nach Anspruch 5, **dadurch gekennzeichnet, dass** die Kugeln (11) beim Gebrauch den Bund (1"') des Stifts (1) aufgrund des Kontakts desselben mit einer versenkten Oberfläche (30) auf den Hohlraum des Griffs (4), der einen kleineren Durchmesser als die innere Aufnahme des Griffs (4) hat, drücken, wobei bei einer stärkeren Druckwirkung auf den Knopf (5) der Schaft (3) teilweise aus dem Griff (4) austritt, so dass die Kugeln (11) in einen Bereich gelangen, wo sie in Kontakt mit einer Oberfläche (31) kommen, die einen größeren Durchmesser als diese Aufnahme hat, wodurch das Lösen des Stifts (1) von dem Schaft (3) aufgrund der Tatsache bedingt wird, dass die Kugeln (11) aus der Aufnahme des Bunds (1'") austreten, und zugleich das Lösen der Tülle (2) vom Griff (4) aufgrund des Austritts der Kugeln (21) aus den Löchern (10) bedingt wird.

## Revendications

1. DISPOSITIF DE PÉDICURE AVEC LAME DE COUPAGE INTERCHANGEABLE comprenant: un manche de forme sensiblement cylindrique (4); une structure de serrage couplée de manière amovible à une extrémité du manche (4), la structure de serrage comprenant une première mâchoire (2) et une deuxième mâchoire (1) pour serrer une lame de coupe ; un arbre (3) adapté pour être inséré dans une cavité centrale du manche (4), l'arbre (3) comprenant un siège (3') à une de ses extrémités ; et un bouton d'actionnement (5) situé à l'extrémité opposée du manche (4) où la structure de serrage est située, le bouton d'actionnement (5) étant adapté pour être couplé à l'arbre (3), dans lequel la première mâchoire (2) comprend une extrémité fuselé et en saillie (2') et un bec (2), dans lequel la deuxième mâchoire (1) a une structure en forme de broche comprenant à une extrémité de celle-ci un profil conjugué (1') à celui de l'extrémité fuselé et en saillie (2') de la première mâchoire (2) et à l'autre extrémité une extrémité à profil en forme de sphère (1") et une zone à profil en collier (1'"), dans lequel l'extrémité de la deuxième mâchoire (1) qui comprend l'extrémité en forme de sphère (1") et la zone à profil en collier (1"') est adaptée pour être insérée dans le siège (3') de l'arbre (3) ; dans lequel une légère action de pression sur le bouton (5) entraîne l'ouverture de la structure de serrage pour permettre un changement de lame de coupe ; et dans lequel une action de pression accrue sur le bouton (5) entraîne la libération de la première mâchoire (2) et la deuxième mâchoire (1) par rapport au manche (4) pour permettre leur nettoyage et leur stérilisation.

2. DISPOSITIF selon la revendication 1, **caractérisé en ce que** deux trous (3"') sont présents au niveau de la paroi latérale du siège (3'), dans chacun desquels est insérée une sphère (11) qui a une liberté de mouvement donnée à l'intérieur du trou dans lequel elle est insérée, étant prévu que, quand l'extrémité (1") de la broche (1) est insérée dans le siège (3), il se produit que les sphères (21) se retrouvent au niveau de la rainure annulaire (1"') de la broche 1, entraînant la fixation de cette dernière avec l'arbre (3).

3. DISPOSITIF selon la revendication 2, **caractérisé en ce que** le bec (2) peut être fixé au manche (4) et, afin de permettre cette fixation, trois autres sphères (21) sont présentes, insérées dans des trous (22) présents à l'extrémité (4') du manche (4) faisant face à la zone de l'outil dans laquelle une lame est présente, étant prévu que, quand le bec (2) est amené en contact avec le manche (4), l'insertion de l'extrémité de surface extérieure des sphères (11) dans les trous (10) présents à l'extrémité du bec se produit, en déterminant ainsi la fixation entre ces deux éléments.

4. DISPOSITIF selon la revendication 3, **caractérisé en ce que** l'extrémité (3"') de l'arbre disposée au niveau de la partie arrière est filetée et un ressort à spirale (6) est disposé autour celle-ci, ladite extrémité filetée (3"') étant adaptée pour être engagée par vissage dans une cavité (5"), également filetée, présente dans le bouton (5), qui a une extrémité extérieure élargie (5').

5. DISPOSITIF selon la revendication 4, **caractérisé en ce que**, en pressant légèrement le bouton (5), la compression du ressort (6) et ainsi le déplacement vers la partie avant de l'arbre (3) se produisent, en résultant un mouvement vers l'avant de la broche (1) par rapport au bec (2), permettant ainsi à la lame d'être libérée de la contrainte entre les deux mâchoires de l'élément de serrage.

6. DISPOSITIF selon la revendication 5, **caractérisé en ce que**, en service, les sphères (11) compriment le collier (1"') de la broche (1), en raison du contact de celles-ci avec une surface évidée (30) sur la cavité du manche (4) qui a un diamètre plus étroit que le siège intérieur du manche (4), dans lequel, lors d'une action de pression accrue sur le bouton (5), la sortie partielle de l'arbre (3) par rapport au manche (4) se produit, de sorte que les sphères (11) se retrouvent dans une zone dans laquelle elles viennent en contact avec une surface (31) ayant un diamètre plus large que ce siège, déterminant la libération de la broche (1) par rapport à l'arbre (3), en raison du fait que les sphères (11) sortent du siège du collier (1"') et déterminant aussi simultanément la libération du bec (2) par rapport au manche (4), en raison du désengagement des sphères (21) par rapport aux trous (10).
